# EUROPÄISCHE PATENTSCHRIFT

(11) **EP 0 532 732 B1**
(45) Veröffentlichungstag und Bekanntmachung des Hinweises auf die Patenterteilung: **07.06.1995**
(21) Anmeldenummer: 92908737.7
(22) Anmeldetag: 24.03.1992
(51) Int. Cl.: A61B 17/22, A61B 6/12

(54) **VORRICHTUNG ZUR ERZEUGUNG VON FOKUSSIERTEN AKUSTISCHEN DRUCK- ODER STOSSWELLEN FÜR THERAPEUTISCHE ANWENDUNGEN MIT EINER RÖNTGEN-ORTUNGSEINRICHTUNG**
DEVICE FOR GENERATING FOCUSSED ACOUSTIC PRESSURE OR SHOCK WAVES FOR THERAPEUTICAL PURPOSES WITH AN X-RAY LOCATING ARRANGEMENT
DISPOSITIF DE GENERATION D'ONDES ACOUSTIQUES FOCALISEES DE PRESSION OU DE CHOC A DES FINS THERAPEUTIQUES AU MOYEN D'UN SYSTEME RADIOGRAPHIQUE DE LOCALISATION

(30) Priorität: 24.03.1991 DE 4109558
(43) Veröffentlichungstag der Anmeldung: 24.03.1993
(73) Patentinhaber: STORZ MEDICAL AG, CH-8280 Kreuzlingen (CH)
(72) Erfinder: HAGELAUER, Ulrich, D-8598 Bottighofen (DE)
(74) Vertreter: Münich, Wilhelm, Dr.
(86) Internationale Anmeldenummer: EP9200651
(87) Internationale Veröffentlichungsnummer: WO9217119

(56) Entgegenhaltungen:
- DE-C- 3 919 083
- DE-C- 4 033 439

## Beschreibung

### Technisches Gebiet

Die Erfindung bezieht sich auf eine Vorrichtung zur Erzeugung von fokussierten akustischen Druck- oder Stoßwellen für therapeutische Anwendungen und insbesondere zur Zerstörung von Konkrementen, Körpersteinen etc., mit einer Druck- bzw. Stoßwellenquelle, einer Ortungseinrichtung für das zu zerstörende Konkrement, die eine Röntgeneinrichtung mit einer Zieleinrichtung aufweist, die die durch die Röntgenstrahlung der Röntgenquelle auf einen Röntgenempfänger projizierbar ist, und einer Patientenaufnahme.

Vorrichtung zur Erzeugung von fokussierten akustischen Druck- oder Stoßwellen für therapeutische Anwendungen und insbesondere zur Zerstörung von Konkrementen, Körpersteinen etc. oder zur Beschallung von Körpergewebe in Lebewesen benötigen Ziel- bzw. Ortungseinrichtungen, um das Konkrement oder Gewebe in dem Fokus der Druckwellenquelle zu positionieren.

### Stand der Technik

Bei bekannten Vorrichtungen werden hierzu eine oder mehrere Röntgenachsen verwendet, die in einer festen räumlichen Zuordnung am Gerät befestigt sind. Ein Fadenkreuz auf dem Monitor markiert den gedachten Durchstoßpunkt einer Geraden von der Röntgenquelle durch den Druckwellenfokus und durch die Empfangsebene. Zur Lokalisierung werden zwei Röntgenaufnahmen mit unterschiedlichen Raumwinkeln benötigt, wobei der Schnittpunkt der beiden Geraden durch den Fokuspunkt (bzw. -bereich) der akustischen Wellen verlaufen muß. Die beiden Durchstrahlungsrichtungen lassen sich durch zwei Röntgenachsen realisieren; alternativ wird eine Röntgenachse mechanisch so bewegt, daß die beschriebene Gerade stets durch den Fokuspunkt verläuft. Für die Zielgenauigkeit wird, je nach Fokusgröße, eine Abweichung von kleiner 1-2 mm verlangt. Wird diese Bedingung nicht erfüllt, so entsteht ein gesundheitliches Risiko für den Patienten, da neben dem Konkrement liegendes Gewebe getroffen wird. Es muß daher ein relativ hoher technischer Aufand betrieben werden, um die erforderliche Präzision der Bewegung zu erreichen.

Weiter sind aus der DE 39 19 083 Al Zieleinrichtungen bekannt, die gemeinsam mit dem interessierenden Körpergebiet auf die Empfangsfläche abgebildet werden. Allerdings ergibt sich bei diesen Zieleinrichtungen das Problem, daß die Röntgenanlage entweder wieder sehr präzise bewegt werden muß (Kimme und Korn auf einer Linie mit dem Brennfleck der Röntgenröhre) oder - wenn eine räumliche Abweichung des Brennflecks zugelassen wird - eine aufwendige Rekonstruktion des Fokuspunktes aus den projizierten Figuren erforderlich ist. Beschrieben werden Lichtgriffel, mit denen der Arzt die Figuren auf dem Monitor markieren muß und eine nachgeschaltete Rechnerauswertung.

Damit erfordert diese bekannte Zieleinrichtung nicht nur einen erhöhten Zeitaufwand für den Arzt, sondern bedingt auch einen relativ hohen technischen Einsatz.

### Beschreibung der Erfindung

Der Erfindung liegt deshalb die Aufgabe zugrunde, eine Vorrichtung zur Erzeugung von fokussierten akustischen Druck- oder Stoßwellen für therapeutische Anwendungen mit einer Röntgen-Ortungseinrichtung derart weiterzubilden, daß der technische und zeitliche Aufwand gegen-Über dem Stand der Technik deutlich reduziert werden kann, ohne daß die Genauigkeit der Ortung verloren geht, und daß insbesondere die erhaltenen Röntgenaufnahmen visuell auswertbar sind.

Eine erfindungsgemäße Lösung dieser Aufgabe ist im Anspruch 1 angegeben, gemäß dem die Vorrichtung dadurch weitergebildet wird, daß die Zieleinrichtung derart ausgebildet ist, daß ihre Projektion auf dem Röntgenempfänger eine vom Ort der Röntgenquelle und des Röntgenempfängers unabhängige Figur ist, und daß die Verbindungslinie zwischen Röntgenquelle und der Mitte der projizierten Figur stets durch einen raumfesten Punkt außerhalb der Zieleinrichtung verläuft.

Weiterbildungen der Erfindung sind Gegenstand der Unteransprüche.

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1: eine Erläuterung des erfindungsgemäßen Grundgedankens,
- Fig. 2: schematisch das sich ergebende Röntgenbild,
- Fig. 3: einen Ausschnitt aus einer erfindungsgemäß ausgebildeten Vorrichtung,
- Fig. 4: eine erfindungsgemäß ausgebildete Vorrichtung mit einer möglichen Patientenaufnahme, und
- Fig. 5 bis 8: verschiedene Ausführungsbeispiele für erfindungsgemäß ausgebildete Zieleinrichtungen.

### Darstellung von Ausführungsbeispielen

In den folgenden Figuren sind jeweils gleiche oder entsprechende Teile mit den selben Bezugszeichen bezeichnet, so daß auf eine erneute Vorstellung verzichtet wird, und lediglich die Abweichungen der in diesen Figuren dargestellten Ausführungsbeispiele gegenüber dem ersten Ausführungsbeispiel erläutert werden:

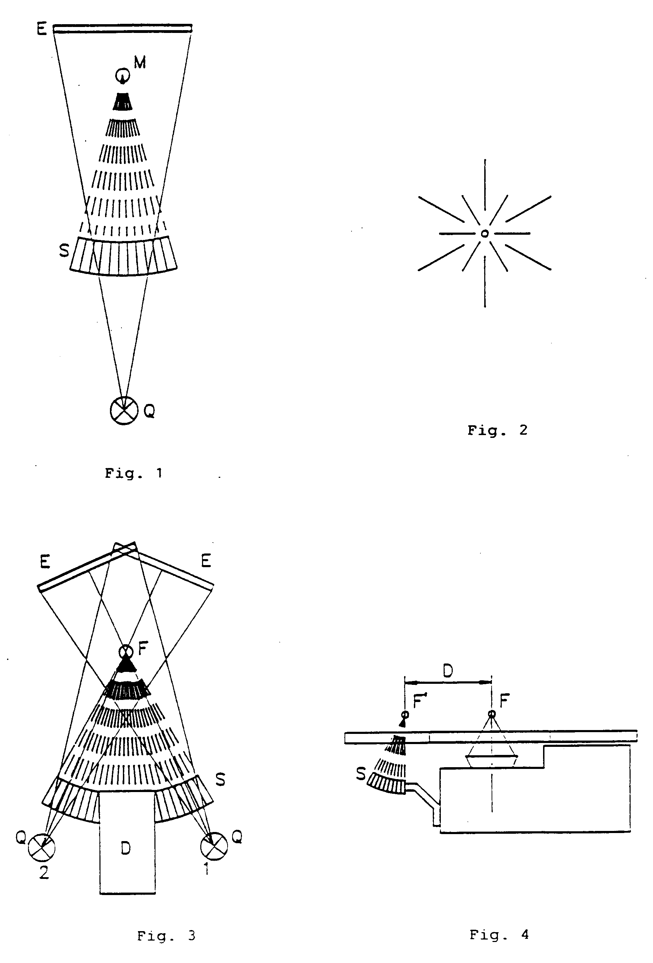

Fig. 1 erläutert den erfindungsgemäßen Grundgedanken. In einer Hohlkugelkalotte S befinden sich entweder Kanäle, wenn das Material der Kalotte S die Strahlung einer Röntgenquelle Q absorbiert, oder Stifte, wenn das Material der Kalotte S röntgentransparent ist. In jedem Falle sind die Bohrungen oder Stifte auf die Kugelmitte M ausgerichtet. Projiziert man eine solche Struktur mit der Röntgenquelle Q auf eine Empfangsfläche E, so ergeben sich Röntgenschatten oder -aufhellungen, wie sie in Fig. 2 dargestellt sind.

Die wesentlichen Eigenschaften der in Fig. 1 exemplarisch dargestellten Struktur S sind, daß sich unabhängig vom Ort der Röntgenquelle Q und der Empfangsfläche E stets ähnliche Abbildungen ergeben, wobei die Verbindungslinie zwischen Röntgenquelle und der Mitte der projizierten Figur stets durch den Kugelmittelpunkt verlaufen. Der technische Aufwand für die präzise Positionierung der Röntgenquelle und des Röntgenempfängers entfällt damit. Ein weiterer Vorteil liegt darin, daß die Figur rein visuell ausgewertet wird, da das Auge das Zentrum leicht als solches erkennt. Das Verfahren nutzt also die dem menschlichen Sehen eigentümliche Fähigkeit zur Mustererkennung und ersetzt damit technische Mittel zur Bildverarbeitung.

Fig. 3 zeigt die Anordnung der in Fig. 1 dargestellten Struktur an einer Druckwellenquelle D, wobei Fokuspunkt F der Druckwellenquelle D und der Kugelmittelpunkt der Kalotte S, auf den die Strukturen ausgerichtet sind, übereinstimmen.

In einer ersten Position 1 der Röntgenquelle wird ein nicht im Fokus liegendes Konkrement zusammen mit der Struktur S durchleuchtet und auf der Empfängerfläche E abgebildet. Der Patient wird daraufhin so verschoben, daß der Konkrementschatten auf die Mitte (Fig. 2) der projizierten Figur zu liegen kommt.

Diese Verschiebung geschieht in einer Ebene parallel zur Empfängerfläche E. In einer zweiten Position 2 der Röntgenquelle wird der Vorgang wiederholt, wobei die Verschiebung diesmal senkrecht zur Empfängerfläche E erfolgt. Nach diesen zwei Schritten befindet sich das Konkrement in Kugelmittelpunkt bzw. Fokus.

Fig. 4 zeigt eine erfindungsgemäße Vorrichtung mit einer Patientenaufnahme, die als verschiebbarer Tisch bzw. als verschiebbare Liege ausgebildet ist, wie er in DE 38 35 317 Al beschrieben ist. Ein solcher Lithotripter erlaubt eine Längsverschiebung der Patientenliege zwischen zwei definierten Punkten. Die Positionierung des Konkrementes erfolgt in den virtuellen Fokus F', der sich in einem definierten Abstand D vom Fokuspunkt F befindet. Die Ortungsstruktur ist so am Grundgerät befestigt, daß sich der Kugelmittelpunkt mit dem Punkt F' deckt. Die Positionierung des Konkrementes erfolgt wie oben beschrieben, wobei nach der Positionierung in F' der Patient nach F verschoben wird. Zur Durchführung von Kontrollaufnahmen ohne die überlagerte Ortungsstruktur läßt sich letztere zur Seite aus dem Strahlengang der Röntgenanlage schieben, abklappen oder dgl.

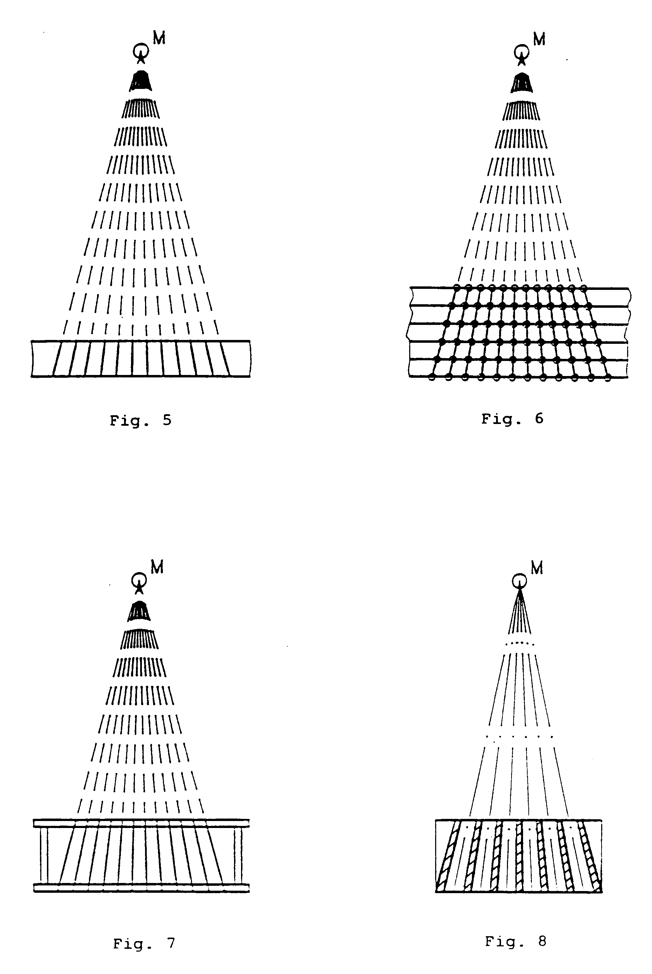

Fig. 5 bis 8 zeigen verschiedene Ausführungen von Ortungsstrukturen bzw. Zieleinrichtungen, die die genannten Eigenschaften aufweisen, und die insbesondere derart ausgebildet sind, daß ihre Projektion auf dem Röntgenempfänger eine vom Ort der Röntgenquelle und des Röntgenempfängers unabhängige Figur ist, und daß die Verbindungslinie zwischen Röntgenquelle und der Mitte der projizierten Figur stets durch einen raumfesten Punkt außerhalb der Zieleinrichtung verläuft.

Fig. 5 zeigt eine ebene Platte, in die Bohrungen, Stifte oder Drähte eingebracht sind, die auf einen Kugelmittelpunkt M ausgerichtet sind.

Fig. 6 zeigt eine Anordnung aus mehreren Platten, in deren Oberfläche röntgenabsorbierende Schichten oder Körper, bzw. die Röntgendurchlässigkeit erhöhende Bohrungen oder Vertiefungen eingebracht sind. Die Strukturen sind wiederum jeweils auf einen Kugelmittelpunkt M zentriert angeordnet.

Fig. 7 zeigt eine Anordnung aus zwei Platten, zwischen denen kugelzentrisch angeordnete Drähte befestigt sind.

Fig. 8 zeigt eine wabenförmige Struktur, deren Schächte auf den Kugelmittelpunkt zentriert sind.

## Patentansprüche

1. Vorrichtung zur Erzeugung von fokussierten akustischen Druck- oder Stoßwellen für therapeutische Anwendungen und insbesondere zur Zerstörung von Konkrementen, Körpersteinen etc., mit
- einer Druck- bzw. Stoßwellenquelle,
- einer Ortungseinrichtung für das zu zerstörende Konkrement, die eine Röntgeneinrichtung mit einer Zieleinrichtung (S) aufweist, die durch die Röntgenstrahlung der Röntgenquelle (Q) auf einen Röntgenempfänger (E) projizierbar ist, und
- einer Patientenaufnahme,
dadurch gekennzeichnet, daß die Zieleinrichtung (S) derart ausgebildet ist, daß ihre Projektion auf dem Röntgenempfanger (E) eine vom Ort der Röntgenquelle (Q) und des Röntgenempfängers (E) unabhängige Figur ist, und daß die Verbindungslinie zwischen Röntgenquelle (Q) und der Mitte der projizierten Figur stets durch einen raumfesten Punkt (M) außerhalb der Zieleinrichtung verläuft.

2. Vorrichtung nach Anspruch 1,
dadurch gekennzeichnet, daß die Zieleinrichtung so an der Druck- bzw. Stoßwellenquelle angeordnet ist, daß sich mindestens zwei Projektionsrichtungen frei wählen lassen, wobei der raumfeste Punkt (M) mit dem Druckwellenfokus (F) übereinstimmt.

3. Vorrichtung nach Anspruch 1,
dadurch gekennzeichnet, daß die Patientenaufnahme zwischen zwei Positionen derart bewegbar ist, daß sich der raumfeste Punkt (M) als ein in einem vorgegebenen Abstand (D) vom Fokuspunkt (F) der Stoß- bzw. Druckwellenguelle liegender Punkt (F') ergibt, auf den die Zieleinrichtung (S) ausgerichtet ist.

4. Vorrichtung nach Anspruch 2,
dadurch gekennzeichnet. daß die Zieleinrichtung (S) eine ebene oder gewölbte Platte aufweist, die wenigstens zwei in Richtung der Röntgenstrahlung nebeneinander angeordnete röntgendurchlässige oder röntgenabsorbierende Strukturen aufweist, und
daß die Strukturen auf den Fokuspunkt (F) der Druck- bzw. Stoßwellenquelle, der außerhalb der Platte liegt, ausgerichtet sind.

5. Vorrichtung nach Anspruch 4,
dadurch gekennzeichnet, daß die röntgendurchlässigen Strukturen Bohrungen und die röntgenabsorbierenden Strukturen Stifte sind.

6. Vorrichtung nach Anspruch 4 oder 5,
dadurch gekennzeichnet, daß wenigstens zwei ebene oder gewölbte Platten vorgesehen sind, von denen röntgendurchlässige oder röntgenabsorbierende Strukturen aufweist, die auf einen einzigen Punkt außerhalb der Platten ausgerichtet sind.

7. Vorrichtung nach Anspruch 6,
dadurch gekennzeichnet, daß zwei ebene oder gewölbte Platten vorgesehen sind, zwischen die röntgenabsorbierenden Stifte bzw. Drähte angeordnet sind.

8. Vorrichtung nach Anspruch 6,
dadurch gekennzeichnet, daß eine Reihe von Platten in Art eines Schichtaufbaus vorgesehen sind.

9. Vorrichtung nach Anspruch 3,
dadurch gekennzeichnet, daß die Zieleinrichtung (S) ebene oder gewölbte Platten mit einer wabenförmigen Struktur aufweist, deren Schächte auf den Punkt (F') außerhalb der Struktur liegenden Fokuspunkt (F) der Druck- bzw. stoßwellenquelle ausgerichtet sind.

10. Vorrichtung nach einem der Ansprüche 6 bis 9,
dadurch gekennzeichnet, daß die gewölbten Platten die Form einer Kugelkalotte haben, und daß der Punkt, auf den die röntgenabsorbierenden bzw. röntgendurchlässigen Strukturen ausgerichtet sind, der Mittelpunkt der zu der Kalotte gehörenden Kugel ist.

## Claims

1. Device for generating focussed acoustic pressure or shock waves for therapeutical purposes, and in particular for destroying concrements, calculi, etc., comprising
- a pressure or shock wave source,
- means for locating the concrement to be destroyed, including X-ray means with sight means (S) which may be projected by the X-radiation of the X-ray source (Q) onto an X-ray receiver (E),
- as well as patient-accommodating means,
**characterized** in that said sight means (S) are so configured that their projection on said X-ray receiver (E) is a figure independent of the sites of said X-ray source (Q) and said X-ray receiver (E), and that the connecting line between said X-ray source (Q) and the centre of the projected figure always passes through a spatially fixed point (M) outside said sight means.

2. Device according to Claim 1,
**characterized** in that said sight means are so disposed on said pressure or shock wave source that at least two directions of projection can be optionally selected, with said spatially fixed point (M) coinciding with the pressure wave focus (F).

3. Device according to Claim 2,
**characterized** in that said patient-accommodating means are adapted to be mobile between two positions in such a way that said spatially fixed point (M) is present as a point (F') spaced from the focus (F) of said shock or pressure wave source, respectively, by a predetermined distance (D), which point (F') said sight means (S) are aligned with.

4. Device according to Claim 2,
**characterized** in that said sight means (S) comprise a plane or arcuate plate including at least two structures transparent to or absorbing X-rays, which are disposed one beside the other in the direction of the X-radiation, and
that said structures are aligned with the focus (F) of said pressure or shock wave source, which is located outside said plate.

5. Device according to Claim 4,
**characterized** in that said structures transparent to X-radiation are bores while said structures absorbing X-rays are pins.

6. Device according to Claim 4 or 5,
**characterized** in that at least two plane or arcuate plates are provided whereof one includes structures transparent to or absorbing X-rays, which are aligned with a single point outside said plates.

7. Device according to Claim 6,
**characterized** in that two plane or arcuate plates are provided between which said X-ray absorbing pins or wires are disposed.

8. Device according to Claim 6,
**characterized** in that several plates are provided in the manner of a laminated structure.

9. Device according to Claim 3,
**characterized** in that said sight means (S) includes plane or arcuate plates having a honey-comb structure with stacks aligned with the point (F') outside the structure, which is spaced from the focus (F) of said pressure or shock wave source.

10. Device according to any of Claims 6 to 9,
**characterized** in that said arcuate plates present the shape of a spherical segment, and that the point which said X-ray absorbing structures or structures transparent to X-radiation are aligned with constitutes the centre of the sphere associated with this spherical segment.

## Revendications

1. Dispositif à engendre des ondes acoustiques focalisées de pression ou de choc à des fins thérapeutiques, et en particulier à détruire des concrétions, calculs, etc., qui comprend
- une source à engendre des ondes de pression ou de choc,
- un moyen à localiser la concrétion à détruire, qui comprend un moyen radiographique à moyen de visée (S) qui peut être projeté par le rayonnement X de ladite source radiogène (Q) sur un récepteur radioscopique (E),
- ainsi qu'un moyen à support-repos de client,
**caractérisé** en ce que ledit moyen de visée (S) est si configuré que sa projection sur ledit récepteur radioscopique (E) est une figure indépendante du lieu de ladite source radiogène (Q) et ledit récepteur radioscopique (E), et en ce que la ligne de jonction entre ladite source radiogène (Q) et le centre de la figure projetée passe toujours par un point fixe en espace (M) au dehors dudit moyen de visée.

2. Dispositif selon la revendication 1,
**caractérisé** en ce que ledit moyen de visée est arrangé à ladite source d'ondes de pression ou de choc, de façon qu'on puisse choisir, à volonté, au moins deux sens de projection, pendant qu'il y a une coïncidence entre ledit point fixe en espace (M) et le foyer (F) de l'onde de pression.

3. Dispositif selon la revendication 2,
**caractérisé** en ce que ledit moyen à support-repos de client est disposé à être déplacé entre deux positions de façon que ledit point fixe en espace (M) est présent comme point (F') espacé du foyer (F) de la source d'ondes de pression ou respectivement de choc, par une distance déterminée (D), sur lequel point (F') est aligné ledit moyen de visée (S).

4. Dispositif selon la revendication 2,
**caractérisé** en ce que ledit moyen de visée (S) comprend une plaque plane ou en arc à au moins deux structures transparentes aux ou absorbantes des rayons X, qui sont disposées l'un de côté de l'autre le long du trajet du rayonnement X, et
en ce que lesdites structures sont alignées sur le foyer (F) de ladite source d'ondes de pression ou respectivement de choc, qui se trouve au dehors de ladite plaque.

5. Dispositif selon la revendication 4,
**caractérisé** en ce que lesdites structures transparentes au rayonnement X sont des creux, pendant que lesdites structures qui absorbent des rayonnement X sont des chevilles.

6. Dispositif selon la revendication 4 ou 5,
**caractérisé** en ce qu'au moins deux plaques planes ou en arc sont prévues, dont l'une comprend des structures qui sont transparentes aux ou qui absorbent des rayons X, et qui sont alignées relativement à un seul point au dehors desdites plaques.

7. Dispositif selon la revendication 6,
**caractérisé** en ce que deux plaques planes ou en arc sont prévues, entre lesquelles lesdits chevilles ou fils métalliques sont disposés, qui absorbent des rayons X.

8. Dispositif selon la revendication 6,
**caractérisé** en ce que plusieurs plaques sont prévues de façon d'une structure stratifiée.

9. Dispositif selon la revendication 3,
**caractérisé** en ce que ledit moyen de visée (S) comprend des plaques planes ou en arc, à structure en nid d'abeilles, qui présente des pans alignés sur le point (F') au dehors de la structure, qui se trouve à une distance du foyer (F) de ladite source d'ondes de pression ou de choc.

10. Dispositif selon une quelconque des revendications 6 à 9,
**caractérisé** en ce que lesdites plaques planes ou en arc ont la forme d'une calotte sphérique, et en ce que le point sur lequel sont alignées lesdites structures à absorber les rayons X, ou les structures transparentes au rayonnement X, constitue le centre de la sphère qui appartient a cette calotte sphérique.
